# EUROPÄISCHE PATENTSCHRIFT

(11) **EP 2 399 984 B1**
(45) Veröffentlichungstag und Bekanntmachung des Hinweises auf die Patenterteilung: **20.03.2013**
(21) Anmeldenummer: 10006458.3
(22) Anmeldetag: 22.06.2010
(51) Int. Cl.: C12M 1/42, C12M 3/00

(54) **Verfahren und Elektrodenanordnung zur Behandlung von adhärenten Zellen**
Method and electrode assembly for treating adherent cells
Procédé et agencement d'électrodes pour le traitement de cellules adhérentes

(43) Veröffentlichungstag der Anmeldung: 28.12.2011
(73) Patentinhaber: Lonza Cologne GmbH, 50829 Köln (DE)
(72) Erfinder: Müller-Hartmann, Herbert, 50937 Köln (DE); Wirth, Andreas, 49525 Lengerich (DE)
(74) Vertreter: Remus, Alvaro Johannes

(56) Entgegenhaltungen:
- US-A1- 2009 305 380
- US-B1- 6 352 853
- Anonymous: "Petri-Pulser PP35-2P - Technical information"[Online] 9. November 2010 (2010-11-09), XP002609839 BTX Harvard Apparatus Holliston (US) Gefunden im Internet: URL:http://www.btxonline.com/products/elec trodes/celltransfection/> [gefunden am 2010-11-09]
- DATABASE WPI Week 200449 Thomson Scientific, London, GB; AN 2004-514495 XP002610220 & JP 2004 202086 A (TEIKOKU SEIYAKU KK) 22. Juli 2004 (2004-07-22)

## Beschreibung

Die Erfindung betrifft eine Elektrodenanordnung, insbesondere zur Beaufschlagung von adhärenten Zellen mit mindestens einem elektrischen Feld, die mindestens zwei Elektroden umfasst, welche jeweils mindestens eine Fläche aufweisen, die der entsprechenden Fläche der jeweils anderen Elektrode gegenüberliegend angeordnet ist, wobei zwischen den Flächen der Elektroden zumindest teilweise ein elektrisch isolierendes Material angeordnet ist. Die Erfindung betrifft ferner ein Verfahren zur Beaufschlagung von adhärenten Zellen mit mindestens einem elektrischen Feld, bei dem das elektrische Feld durch das Anlegen einer Spannung and mindestens zwei Elektroden erzeugt wird.

Die Beaufschlagung von lebenden Zellen mit einem elektrischen Feld bzw. Spannungsimpuls, die so genannte Elektroporation bzw. Elektrotransfektion, wird seit Jahren auf Zellen in den verschiedensten Zuständen angewandt. Als Einzelzellen in Suspension in einer Pufferlösung, im adhärenten Zustand in einem Kulturgefäß, meist am Boden eines Kunststoffbehälters und in vivo, wo Zellen in der Regel im Gewebeverband in eine extrazelluläre Matrix eingebettet sind. Grundsätzlich werden bei der Elektroporation die Fremdmoleküle aus einer an die Zellen angepassten Pufferlösung oder einem Zellkulturmedium durch einen kurzzeitigen Stromfluss in die Zellen eingebracht, wobei durch die Wirkung der elektrischen Spannungsimpulse bzw. des dadurch entstehenden elektrischen Feldes und Stromflusses die Zellmembran für die Fremdmoleküle durchlässig gemacht wird. Die Zellsuspension befindet sich dabei häufig in einer sogenannten Küvette, d.h. einem schmalen, offenen Gefäß, dessen Probenraum zwei gegenüberliegende, parallele Elektroden in den Seitenwänden aufweist, welche zum Anlegen der elektrischen Spannung dienen. Durch die kurzzeitig entstehenden "Poren" in der Zellmembran gelangen die biologisch aktiven Moleküle zunächst in das Zytoplasma, in dem sie ggf. bereits ihre zu untersuchende Funktion ausüben können, und daraufhin unter bestimmten Bedingungen auch in den Zellkern. Durch das kurzzeitige Anlegen eines starken elektrischen Feldes, d.h. eines kurzen Spannungsimpulses mit hoher Stromdichte, können darüber hinaus auch Zellen, Zellderivate, subzelluläre Partikel und/oder Vesikel fusioniert werden. Bei dieser sogenannten Elektrofusion werden die Zellen beispielsweise zunächst durch ein inhomogenes elektrisches Wechselfeld in engen Membrankontakt gebracht. Durch das anschließende Anlegen eines elektrischen Feldimpulses kommt es dann zur Interaktion von Membranteilen, die schließlich zur Fusion führt. Für die Elektrofusion können dabei vergleichbare apparative Vorrichtungen verwendet werden, wie für die Elektroporation. Darüber hinaus können lebende Zellen durch elektrische Felder auch in einer ihre Eigenschaften verändernden Weise stimuliert werden.

Aus der WO 2005/056788 A1 ist beispielsweise ein Verfahren zur Elektroporation bekannt, bei dem Zellen auf einer mikroporösen Membran wachsen, die sich zwischen zwei parallel angeordneten Elektrodenflächen befindet.

Die US-A-5 134 070 beschreibt Anwendungen und Vorrichtungen zur Elektroporation von Zellen, die auf einer elektrisch leitenden Oberfläche wachsen, welche als Elektrode dient. Das Kulturgefäß wird von oben mit einer plattenförmigen Gegenelektrode abgedeckt, wobei ein Spalt gebildet wird, über den elektrische Entladungen möglich sind.

Aus der WO 2008/104086 A1 ist ferner eine Vorrichtung bekannt, bei der die Zellen auf co-planaren Elektrodenflächen wachsen. Der elektrische Kontakt zwischen den Elektroden wird über das Zellkulturmedium über den Zellen hergestellt, wobei die zwei Elektrodenbereiche durch eine isolierende Barriere getrennt sind, die aber trotzdem eine Elektrolytbrücke zwischen den Elektroden zulässt. Diese können beispielsweise aus Indiumzinnoxid bestehen, das als transparenter Halbleiter eine mikroskopische Analyse der Zellen ermöglicht.

Aus der WO 2009/131972 A1 ist eine Vorrichtung zur Elektroporation von Zellen bekannt, welche adhärent auf einer runden, scheibenförmigen Platte wachsen. Die Vorrichtung weist zwei parallel zueinander angeordnete Elektroden auf, wobei eine Elektrode sich auf der konkaven Oberfläche eines außen liegenden Zylinders und die andere Elektrode sich auf der konvexen Oberfläche eines innen liegenden Zylinders befindet.

Aus der US 2009/0305380 A1 ist femer eine Vorrichtung zur Elektroporation von Zellen bekannt, die auf einer festen Fläche immobilisiert sind. Das elektrische Feld, mit dem die Zellen beaufschlagt werden, wird durch eine Anordnung von Elektrodenpaaren erzeugt, die sich dicht nebeneinander liegend auf einer oberhalb der festen Fläche angeordneten Oberfläche befinden. Die Elektroden werden durch elektrische Bahnen gebildet, die auf die Oberfläche aufplattiert sind. Die beiden Elektroden eines Elektrodenpaars sind dabei so dicht nebeneinander angeordnet, dass sich nicht mehr als eine Zelle innerhalb des geringsten Abstandes zwischen den beiden Elektroden befinden kann.

Die US-A-6 352 853 beschreibt kammförmige Elektrodenanordnungen für die Elektrotransfektion von Zellen, die in Form einer Matrix angeordnet werden, welche eine Vielzahl von Elektrodenpaaren umfasst. Jedes einzelne Elektrodenpaar, das von zwei nebeneinander liegenden Elektroden benachbarter Elektrodenanordnungen gebildet wird, taucht dabei in eine Vertiefung einer Multiwell-Platte ein. Die beiden Elektroden eines Elektrodenpaars werden durch einen Abstandhalter aus nicht-leitendem Material teilweise voneinander getrennt.

Die Firma BTX vertreibt mit dem PetriPulser^{®} eine Anordnung von abwechselnd gepolten planparallelen Elektrodenplatten, die senkrecht auf adhärent in einem Kulturgefäß wachsende Zellen aufgebracht werden kann. Dabei tauchen die Elektroden in den Kulturüberstand ein, wobei sich die Zwischenräume zwischen den einzelnen Elektrodenplatten mit dem Kulturmedium füllen. Ein wesentlicher Nachteil dieser Anordnung liegt darin, dass der Großteil des Stromes über das über den Zellen befindliche zellfreie Kulturmedium abfließt. Das Feld ist aber nur wirksam im Randfeld am Boden des Gefäßes, wo sich die Zellen befinden, so dass unnötig hohe Ströme zu erbringen sind. Ferner muss von einer hohen Mortalität der Zellen durch pH-Wert-Änderungen und hohen Strom ausgegangen werden. Darüber hinaus muss die Spannungsversorgung für lang anhaltende Spannungsimpulse sehr groß ausgelegt werden, um diese großen Ströme und damit Ladungsmengen und Leistungen zu erbringen. Zudem muss ein großes Volumen aufgebracht werden, das für die Elektroporation geeignet ist und das zu transfizierende Substrat in ausreichend hoher Konzentration enthält, wodurch auch die Menge an Substrat entsprechend höher liegt.

Der Erfindung liegt die Aufgabe zugrunde, eine Elektrodenanordnung und ein Verfahren zu schaffen, die eine effiziente Behandlung von adhärenten Zellen mit einem elektrischen Feld ermöglichen, ohne zu hohe Stromdichten zu benötigen.

Die Aufgabe wird erfindungsgemäß durch eine Elektrodenanordnung der eingangs genannten Art gelöst, bei der die Flächen der Elektroden durch das elektrisch isolierende Material vollständig voneinander getrennt sind. Durch diese erfindungsgemäße Lösung wird erreicht, dass das elektrische Feld im Bereich der zu behandelnden Zellen konzentriert werden kann, so dass ein Spannungsimpuls bzw. der hierdurch entstandene Strom durch die Zellen fließt, ohne dass ein Hauptteil davon ungenutzt im Elektrolyten über den Zellen abfließt. Hierdurch kann einerseits die Vorrichtung zur Impulserzeugung sparsam dimensioniert werden und andererseits können in dem Medium stärkere Veränderungen des pH-Wertes vermieden werden, die ansonsten durch große geflossene Ladungsmengen infolge von Elektrolyse erzeugt würden. Durch die erfindungsgemäße Vorrichtung wird femer gewährleistet, dass eine räumlich möglichst gleichmäßig verteilte elektrische Behandlung über die Kulturfläche erfolgt und Bereiche mit nicht-behandelten Zellen minimiert werden. Dabei sind der prozentuale Anteil von erfolgreich behandelten (beispielsweise transfizierten) Zellen und die Überlebensrate sowie, bei Verwendung von DNA oder mRNA, das Expressionsniveau pro Zelle vergleichbar mit den entsprechenden Werten bei der Elektroporation von Zellen in Suspension. Mittels der erfindungsgemäßen Elektrodenanordnung wird also eine effiziente Behandlung von adhärenten Zellen mit einem elektrischen Feld ermöglicht.

In vorteilhafter Ausgestaltung der erfindungsgemäßen Elektrodenanordnung ist vorgesehen, dass diese mindestens drei, vorzugsweise mindestens 4 oder 5, insbesondere 6 bis 12, Elektroden umfasst.

Wenn die Elektroden platten- oder stiftförmig ausgebildet sind, können möglichst viele Elektroden auf engem Raum angeordnet werden, so dass ein besonders homogenes elektrisches Feld erzeugt werden kann. In alternativer Ausgestaltung der Erfindung können also Plattenelektroden durch Reihen von Metallstiften ersetzt werden. Wenn diese Reihen von elektrisch gekoppelten Stiften ausreichend eng angeordnet sind, können sie bezüglich des erzeugten elektrischen Feldes durchgehende Plattenelektroden ersetzen. "Ausreichend eng" heißt in diesem Zusammenhang, dass der Abstand gleich gepolter benachbarter Stifte kleiner oder maximal gleich dem Abstand der entgegengesetzt gepolten Reihen von Stiften ist. Die Verwendung einer solchen Anordnung ist besonders vorteilhaft, da die Herstellung von Elektrodenanordnungen durch Einlegen von Metallstiften oder Drähten bzw. Drahtstücken in ein Spritzgusswerkzeug und anschließende Umspritzung, beispielsweise mit einem thermoplastischen Polymer, weit verbreitet und daher das Fertigungsverfahren von vielen Herstellern gut beherrschbar ist.

In weiterer vorteilhafter Ausgestaltung der erfindungsgemäßen Elektrodenanordnung ist vorgesehen, dass die Flächen Seitenflächen planparallel angeordneter Elektrodenplatten sind.

Die vollständige Trennung der Flächen durch das isolierende Material wird in vorteilhafter Weise vorzugsweise dadurch erreicht, dass der von den Flächen der Elektroden gegrenzte Raum zwischen den Elektroden vollständig von dem isolierenden Material ausgefüllt ist.

Das isolierende Material ist in vorteilhafter Ausgestaltung der Erfindung ein thermoplastisches Polymer, vorzugsweise Polyvinylchlorid, Polystyrol, Polypropylen, Polyethylen und/oder Polykarbonat. Die Elektroden bestehen vorzugsweise aus Metall und/oder einem elektrisch leitenden Kunststoff.

In vorteilhafter Ausgestaltung der erfindungsgemäßen Elektrodenanordnung ist vorgesehen, dass die Elektrodenanordnung an mindestens einer den Zellen zugewandten Seite mindestens einen Abstandhalter aufweist, der verhindert, dass die Elektroden unmittelbar mit den Zellen in Berührung kommen. Durch den oder die Abstandhalter wird gewährleistet, dass ein Mindestabstand zwischen den Elektroden und den Zellen eingehalten wird und/oder ein definierter Abstand zwischen den Elektroden und den Zellen eingestellt werden kann.

In besonders vorteilhafter Ausgestaltung der erfindungsgemäßen Elektrodenanordnung ist vorgesehen, dass die Elektrodenanordnung zum Einsetzen in mindestens ein zumindest teilweise mit Flüssigkeit gefülltes Gefäß vorgesehen ist, vorzugsweise ein Gefäß, an dessen Bodenfläche lebende Zellen anhaften, und dass das isolierende Material beim Einsetzen in das Gefäß zumindest einen Teil der Flüssigkeit verdrängt. Hierdurch können die Elektroden nah an die zu behandelnden Zellen gebracht und das Volumen der Flüssigkeit, die sich über den Zellen befindet, minimiert werden.

Die Elektroden sind vorzugsweise zumindest teilweise an der Unterseite einer Halterung angeordnet. Diese Halterung kann beispielsweise derart ausgebildet sein, dass sie in ein Reaktionsgefäß eingesetzt oder auf dieses aufgesetzt werden kann, so dass die Elektroden mit dem Innenraum des Reaktionsgefäßes in Kontakt stehen. Das Reaktionsgefäß kann dabei beispielsweise eine einzelne Küvette oder Zellkulturschale oder vorzugsweise Teil einer Multiwell-Platte sein. Die erfindungsgemäße Elektrodenanordnung wird vorzugsweise zum Beaufschlagen von adhärenten Zellen mit mindestens einem elektrischen Feld, insbesondere zur Elektroporation von adhärenten Zellen, vorzugsweise in Form mindestens einer Eintauchelektrodenvorrichtung, verwendet. Die erfindungsgemäße Elektrodenanordnung in Form einer Eintauchelektroden-vorrichtung ermöglicht in vorteilhafter Weise die Transfektion von adhärent wachsenden Zellen, wobei die Elektrodenvorrichtung vor und nach der Transfektion aus dem Medium entfernbar ist. Dabei kann auf einfache Weise die maximale Flexibilität in Bezug auf das verwendete Zellkultursystem sichergestellt werden, insbesondere die Kompatibilität mit möglichst vielen Kultursystemen.

Die Aufgabe wird erfindungsgemäß ferner durch ein Verfahren der eingangs genannten Art gelöst, bei dem das elektrische Feld an der den Zellen zugewandten Seite der Elektroden dadurch konzentriert und/oder auf den Raum zwischen den Zellen und der den Zellen zugewandten Seite der Elektroden begrenzt wird, dass elektrisch isolierendes Material zwischen den Elektroden platziert wird, welches die einander zugewandten Flächen der Elektroden vollständig voneinander trennt. Durch diese erfindungsgemäße Lösung wird erreicht, dass ein Spannungsimpuls bzw. der hierdurch entstandene Strom durch die Zellen fließt, ohne dass ein Hauptteil davon ungenutzt im Elektrolyten über den Zellen abfließt. Hierdurch kann einerseits die Vorrichtung zur Impulserzeugung sparsam dimensioniert werden und andererseits können in dem Medium stärkere Veränderungen des pH-Wertes vermieden werden, die ansonsten durch große geflossene Ladungsmengen infolge von Elektrolyse erzeugt würden. Durch die erfindungsgemäße Vorrichtung wird femer gewährleistet, dass eine räumlich möglichst gleichmäßig verteilte Transfektion über die Kulturfläche erfolgt und Bereiche mit nicht-transfizierten Zellen minimiert werden. Dabei sind der prozentuale Anteil von transfizierten Zellen und die Überlebensrate sowie, bei Verwendung von DNA, mRNA, siRNA oder anderen exprimierbaren Nukleinsäuren, der Grad der Beeinflussung der Expression pro Zelle vergleichbar mit den entsprechenden Werten bei der Elektroporation von Zellen in Suspension. Mittels des erfindungsgemäßen Verfahrens wird also eine effiziente Behandlung von adhärenten Zellen mit einem elektrischen Feld ermöglicht.

In vorteilhafter Ausgestaltung des erfindungsgemäßen Verfahrens ist vorgesehen, dass das elektrische Feld auf den Raum zwischen den Zellen und einer freiliegenden Stirnseite der Elektroden begrenzt wird.

In vorteilhafter Ausgestaltung des erfindungsgemäßen Verfahrens ist femer vorgesehen, dass die Elektroden mit einer freiliegenden Stirnseite in mindestens ein Gefäß eingebracht werden, an dessen Bodenfläche die Zellen anhaften.

In besonders vorteilhafter Ausgestaltung des erfindungsgemäßen Verfahrens ist vorgesehen, dass die Wirkung des elektrischen Feldes auf die Zellen durch Einstellen des Abstands zwischen den Zellen und den Elektroden optimiert wird. Auf diese Weise entsteht über den zu behandelnden Zellen ein homogenes und ausreichend starkes elektrisches Feld, was sich sehr positiv auf die Effizienz der Behandlung auswirkt. So kann beispielsweise die Transfektionseffizienz bei der Elektrotransfektion von Zellen durch Einstellen des Abstandes zwischen den Elektroden und den Zellen optimiert werden.

Die Erfindung wird im Folgenden anhand der Abbildungen beispielhaft näher erläutert.
Figur 1 zeigt (a) eine schematische Seitenansicht einer Elektrodenanordnung gemäß dem Stand der Technik, (b) eine beispielhafte schematische Seitenansicht einer erfindungsgemäßen Elektrodenanordnung und (c) eine schematische Draufsicht auf die Unterseite einer weiteren beispielhaften Ausführungsform einer erfindungsgemäßen Elektrodenanordnung.
Figur 2 zeigt fluoreszenzmikroskopische Bilder der Expression von grün fluoreszierendem Protein (GFP) in HeLa-Zellen, die (a) zum einen mit einer Elektrodenanordnung gemäß dem Stand der Technik und (b) zum anderen mittels einer erfindungsgemäßen Elektrodenanordnung behandelt wurden.
Figur 3 zeigt Balkendiagramme des Vergleichs einer Elektrodenanordnung gemäß dem Stand der Technik (St. d. T.) mit einer erfindungsgemäßen Elektrodenanordnung (erfind. Vorr.), wobei (a) zum einen der Anteil transfizierter Zellen und (b) zum anderen die Überlebensrate der Zellen jeweils in Prozent dargestellt sind (AD-035 = Nummer der elektrischen Parameter für adhärente Zellen, Nucleofector^{®}, Lonza).
Figur 4 zeigt eine perspektivische Ansicht der Unterseite einer beispielhaften Ausführungsform einer erfindungsgemäßen Elektrodenanordnung.
Figur 5 zeigt eine weitere perspektivische Ansicht der Elektrodenanordnung gemäß Figur 4, wobei in dieser Darstellung die innen liegenden Teile der Elektroden und die Kontaktelemente sichtbar sind.
Figur 6 zeigt eine perspektivische Ansicht der Oberseite der Elektrodenanordnung gemäß Figur 4.
Figur 7 zeigt einen Längsschnitt durch die Elektrodenanordnung gemäß den Figuren 4 bis 6.
Figur 8 zeigt in einem Balkendiagramm die Abhängigkeit der Transfektionseffizienz vom Abstand der Elektroden einer erfindungsgemäßen Elektrodenanordnung zu den auf der Kulturfläche anhaftenden Zellen bei drei unterschiedlich starken Spannungsimpulsen (x-Achse: Abstand [mm], γ-Achse: Transfektionseffizienz [%], A-5 = schwacher Spannungsimpuls, K-19 = mittelstarker Spannungsimpuls, AX-19 = starker Spannungsimpuls).

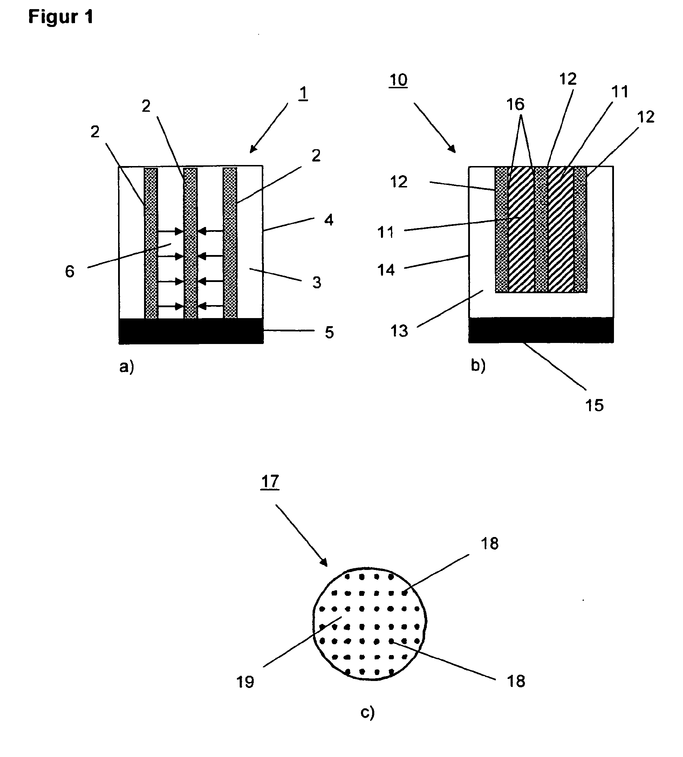

Figur 1 zeigt (a) eine schematische Seitenansicht einer Elektrodenanordnung 1 gemäß dem Stand der Technik mit freiliegenden Elektroden 2 und (b) eine beispielhafte schematische Seitenansicht einer erfindungsgemäßen Elektrodenanordnung 10 mit elektrisch isolierendem Material 11 zwischen den Elektroden 12. Die Elektrodenanordnung 1 gemäß dem Stand der Technik, die im Prinzip dem PetriPulser^{®} der Firma BTX entspricht, besteht aus drei planparallel angeordneten Elektroden 2, die in den Innenraum 3 eines Gefäßes 4 hineinragen und auf der Bodenfläche 5 des Gefäßes 4 aufliegen (Figur 1 a). Auf der Bodenfläche 5 können lebende Zellen anhaften und wachsen (adhärente Zellen). Der Innenraum 3 ist mit einer Flüssigkeit gefüllt, beispielsweise einem Zellkulturmedium oder einer anderen an die Zellen adaptierten Lösung, wobei diese Flüssigkeit auch den freien Raum 6 zwischen den Elektroden 2 ausfüllt. Jede Elektrode 2 ist also vollständig von der Flüssigkeit umgeben. Da die Flüssigkeit elektrisch leitend ist, fließt beim Anlegen einer Spannung an die Elektroden 2 ein Großteil des Stroms über die Flüssigkeit zwischen den Elektroden 2 ab (siehe Pfeile), so dass bei der Verwendung einer nicht-permanenten Spannungsquelle, d. h. beispielsweise der Entladung eines Kondensators, die Spannung sehr schnell abfällt und somit das elektrische Feld über die Zeit geschwächt ist. Nur ein Teil des Stroms fließt über die Bodenfläche 5, so dass die biologische Wirkung des Stromflusses gering ist.

Die erfindungsgemäße Elektrodenanordnung 10 umfasst drei planparallel angeordneten Elektroden 12, die in den Innenraum 13 eines Gefäßes 14 hineinragen (Figur 1 b). Das Gefäß 14 umfasst eine Bodenfläche 15, auf der lebende Zellen anhaften und wachsen können (adhärente Zellen). Der Innenraum 13 ist mit einer Flüssigkeit gefüllt, beispielsweise einem Zellkulturmedium oder einer anderen an die Zellen adaptierten Lösung. Der Raum zwischen den Elektroden 12 ist vollständig mit einem elektrisch isolierenden Material 11 ausgefüllt, so dass beim Anlegen einer Spannung an die Elektroden 12 kein Strom über den zwischen den Elektroden 12 liegenden Raum abfließen kann. Der gesamte Strom fließt bei der erfindungsgemäßen Elektrodenanordnung 10 durch den Raum zwischen den Elektroden 12 und der Bodenfläche 15, so dass hier bei der Verwendung einer nicht-permanenten Spannungsquelle (z. B. Kondensator) die Spannung langsamer abfällt und somit die Feldstärke für die Behandlung der Zellen über die Zeit höher ist. Hierdurch kann einerseits die Vorrichtung zur Impulserzeugung sparsam dimensioniert werden und andererseits können in der Flüssigkeit stärkere Veränderungen des pH-Wertes vermieden werden, die ansonsten durch große geflossene Ladungsmengen infolge von Elektrolyse erzeugt würden.

Erfindungsgemäß können also beispielsweise planparallele Elektroden 12 durch isolierendes Material 11 getrennt sein, so dass die leitenden Oberflächen der Elektroden 12 nur nach unten (in Richtung der Bodenfläche 15 bzw. der darauf haftenden Zellen) frei sind und mit der Umgebung in elektrischem Kontakt stehen. Durch die volle Ausdehnung des isolierenden Materials 11 im Bereich zwischen den jeweils gegenüberliegend angeordneten Flächen 16 der planparallelen Elektroden 12, oder zumindest in dem für die Flüssigkeit offenen Bereich zwischen Elektroden, in dem diese parallele Linien beschreiben, ist das elektrische Feld fokussierbar bzw. der Strom auf den angestrebten Wirkbereich begrenzbar. Es ist femer von besonderem Vorteil, dass eine Fokussierung des elektrischen Feldes im Bereich der adressierten Zellen bzw. eine Begrenzung des elektrischen Stromes auf den Wirkbereich nun auch bei Verwendung von planparallelen Elektroden 12 möglich ist, was über die Zeit konstante und stabilere Feldstärken und Stromdichten in dem adressierten Bereich zwischen den Elektroden 12 und der Bodenfläche 15 ermöglicht. Geeignete isolierende Materialien hierfür sind beispielsweise Platten oder Formspritzkörper aus gängigen, vorzugsweise thermoplastischen, Kunststoffen, wie beispielsweise Polyvinylchlorid, Polystyrol, Polypropylen, Polyethylen oder Polykarbonat. Durch den erfindungsgemäßen Aufbau kann der Abfluss von Strom über die sich jeweils gegenüberliegenden Flächen 16 der planparallelen Anteile der Elektroden 12 verhindert und somit Spannungsimpulse mit konstantem Strom erzeugt werden. Eine erfindungsgemäße Anordnung kann also beispielsweise pro Reaktion, je nach Fläche des Kulturbodens mit den zu behandelnden Zellen, mit einer oder mehreren aufeinander folgenden Impulsentladungen geringerer Energie/Ströme beschickt werden, um die nötigen Leistungen pro Entladung zu begrenzen.

Es kann beispielsweise ein Elektroden-Isolator-Sandwich verwendet werden, bei dem die Elektroden abwechselnd gepolt sind. Bei einer solchen Anordnung ist das Feld in den Bereichen unterhalb der aktiven Elektroden praktisch nicht vorhanden und daher nicht auf die dort vorzufindenden Zellen wirksam, die unterhalb der aktiven Elektroden liegen. Diese Bereiche befinden sich in unmittelbarer Nähe eines elektrischen Leiters (der Elektroden) und daher außerhalb eines nennenswerten Feldes. Daher sollten die Elektroden möglichst dünn sein (beispielsweise 50pm) und die annähernd gesamte zellbewachsene Bodenfläche von der Elektrodenanordnung mit aktiven Bereichen aus Elektroden-Isolator-Kombinationen bedeckt sein. Aktive Bereiche sind hier die Bereiche unterhalb des isolierenden Materials zwischen entgegengesetzt gepolten Elektroden. Hierdurch sind insbesondere im Querschnitt runde Geometrien der Elektrodenanordnung vorteilhaft, die so dimensioniert sind, dass sie in gängige Zellkulturgefäße nach ANSI-SBS-Standard (American National Standards Institute - Society for Biomolecular Sciences) passen.

Figur 1c zeigt eine schematische Draufsicht auf die Unterseite einer weiteren beispielhaften Ausführungsform einer erfindungsgemäßen Elektrodenanordnung 17 mit stiftförmigen Elektroden 18. Da die Elektroden 18 jeweils einen runden Querschnitt aufweisen, umfasst praktisch deren jeweilige gesamte Umfangsfläche Flächen, die den entsprechenden Flächen der anderen Elektroden 18 gegenüberliegend angeordnet sind. Der Raum zwischen den stiftförmigen Elektroden 18 ist bei dieser Ausführungsform daher vollständig mit einem elektrisch isolierenden Material 19 ausgefüllt, so dass nur die Stirnflächen der Elektroden 18 an deren Unterseite frei liegen und mit der Umgebung in elektrischem Kontakt stehen. Alle Elektroden 18 sind folglich jeweils auf ihrer gesamten Umfangsfläche elektrisch gegeneinander isoliert, so dass kein Strom über den Raum zwischen den Elektroden 18 abfließen kann. Der gesamte Strom fließt also auch bei der erfindungsgemäßen Elektrodenanordnung 17 durch den Raum zwischen den Elektroden 18 und den (hier nicht sichtbaren) Zellen, so dass bei der Verwendung einer nicht-permanenten Spannungsquelle (z. B. Kondensator) die Spannung nur langsam abfällt und somit die Feldstärke für die Behandlung der Zellen über die Zeit sehr hoch ist.

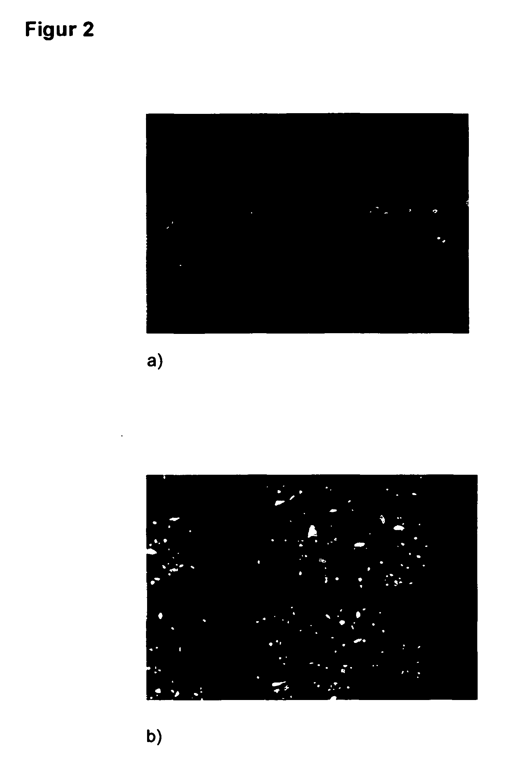

Zu Versuchszwecken wurde eine erfindungsgemäße Vorrichtung bzw. Elektrodenanordnung aus wechselnden Lagen von Aluminiumfolien und 2 mm-Isolatormaterial erstellt. Die verklebte Vorrichtung wurde als Anpassung an die runden Geometrien der Kulturgefäße (6-well, 12-well, 24-well) abgeschliffen und am oberen Ende durch elektrisches Verbinden jeweils jeder zweiten Elektrode auf zwei elektrische Anschlüsse verschaltet. Im Anschluss wurde die Vorrichtung entweder an einer horizontalen Linearschiene befestigt oder direkt händisch in eine Kulturvertiefung eingeführt, in der adhärent Zellen (hier HeLa-Zellen) wachsen. Der Einfachheit halber wurde die Versuchsvorrichtung in diesem Fall mit den Elektroden auf den Kulturboden aufgesetzt, so dass von einem Abstand zwischen Zellen und Elektrodenaufbau von <1 mm ausgegangen werden kann. Das Kulturmedium in dem Gefäß wurde zuvor durch 1 ml einer Mischung von Lösungen (Nucleofector^{®} Cell Line Solution R, Lonza) mit Plasmid-DNA (pmaxGFP^{®}, Lonza, 2 µg/100 µl) ersetzt. Die alternierend zusammen geschalteten Elektrodenfolien wurden daraufhin über einen Nucleofector^{Ⓡ} der Firma Lonza mit verschiedenen Testimpulsen beschickt, die im Bereich von Pulsen liegen, die auch bei der Verwendung von Einzelküvetten mit 100 µl Volumen zur Anwendung kommen. Im Anschluss wurde die Vorrichtung aus der Kulturvertiefung wieder entfernt und das Elektrolyt wieder durch Medium ersetzt, um die Zellen weiter kultivieren zu können. Der Einfachheit halber wurde das Lösung-DNA-Gemisch in verschiedenen Vertiefungen wieder verwendet. Analog wurde mit dem PetriPulser^{Ⓡ} der Firma BTX verfahren, nur dass aufgrund der fehlenden Isolatoren zwischen den Elektroden zur Erzeugung eines vergleichbaren Füllstandes nach Einführen der Elektrodenvorrichtung 2 ml des gleichen Lösung-DNA-Gemischs eingefüllt wurde. Die Zellen wurden nach einem Tag mittels Durchflusszytometrie analysiert. Bei Verwendung des PetriPulsers^{®} der Firma BTX mit annähernd gleichem Elektrodenabstand, also gleichen Voraussetzungen für die Generierung des elektrischen Feldes, sind nur sehr sporadisch überhaupt transfizierte Zellen zu finden (Figur 2a). Darüber hinaus wurden hier Fehlermeldungen zur Überstromabschaltung beobachtet, was einen Hinweis darauf liefert, dass der PetriPulser^{Ⓡ}aufgrund des nicht begrenzten Stromflusses durch offene Zwischenräume zwischen den Elektrodenplatten für die Erzeugung ausreichend hoher elektrischen elektrischer Felder nicht geeignet ist. Dagegen konnten unter Verwendung der erfindungsgemäßen Vorrichtung 30 - 45 % der Zellen transfiziert werden (GFP-Expression, Figur 2b). Es ist also klar ersichtlich, dass der erfindungsgemäße Aufbau adhärente Zellen effizient transfizieren kann.

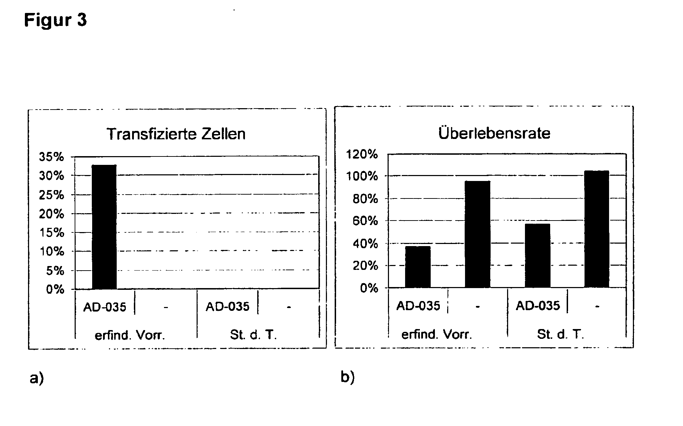

Im Anschluss wurden die Zellen auf Überleben, Morphologie und die Expression der eingebrachten genetischen Information untersucht. Fig. 3 zeigt einen Vergleich von Zellen, die zum einen mittels der zu Figur 2b beschriebenen erfindungsgemäßen Vorrichtung und zum anderen mit dem PetriPulser^{Ⓡ} der Firma BTX entsprechend dem zu Figur 2a beschriebenen Verfahren transfiziert werden. Es konnte gezeigt werden, dass die Zellen mit der erfindungsgemäßen Elektrodenanordnung mit hoher Effizienz und unter Wahrung einer hohen Viabilität und morphologischen Integrität transfizierbar waren (Figuren 3a und 3b). Die Ergebnisse lagen im von der Größenordnung her gleichen Bereich wie Vergleichsdaten mit existierenden Protokollen für diese Zellen bei der Transfektion in Suspension. Nach der Behandlung mit dem PetriPulser^{Ⓡ} konnte zwar eine etwas höhere Überlebensrate festgestellt werden, jedoch keine nennenswerte Transfektion (Figuren 3a und 3b).

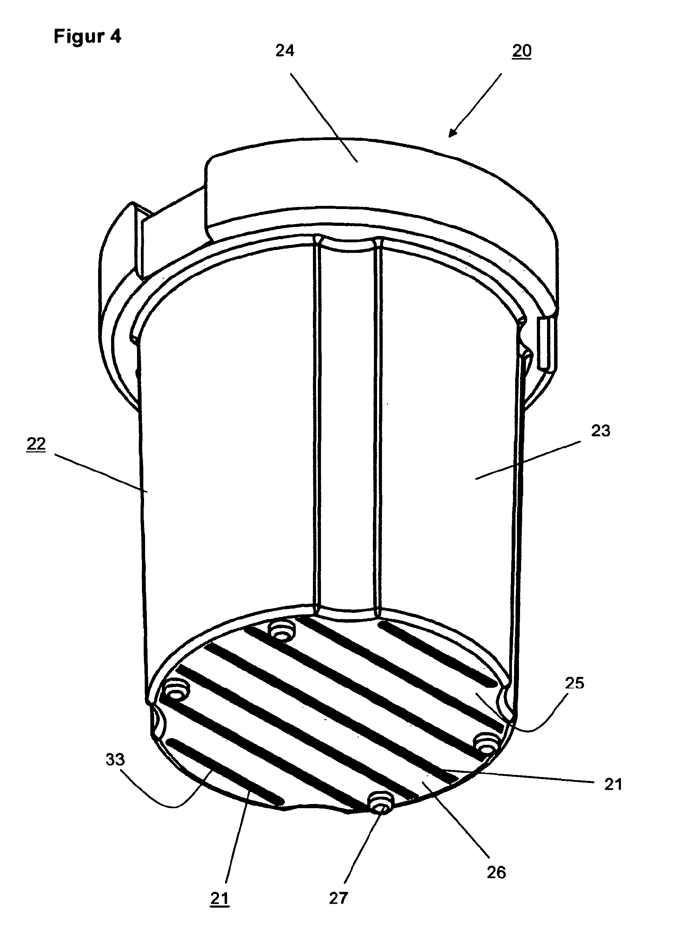

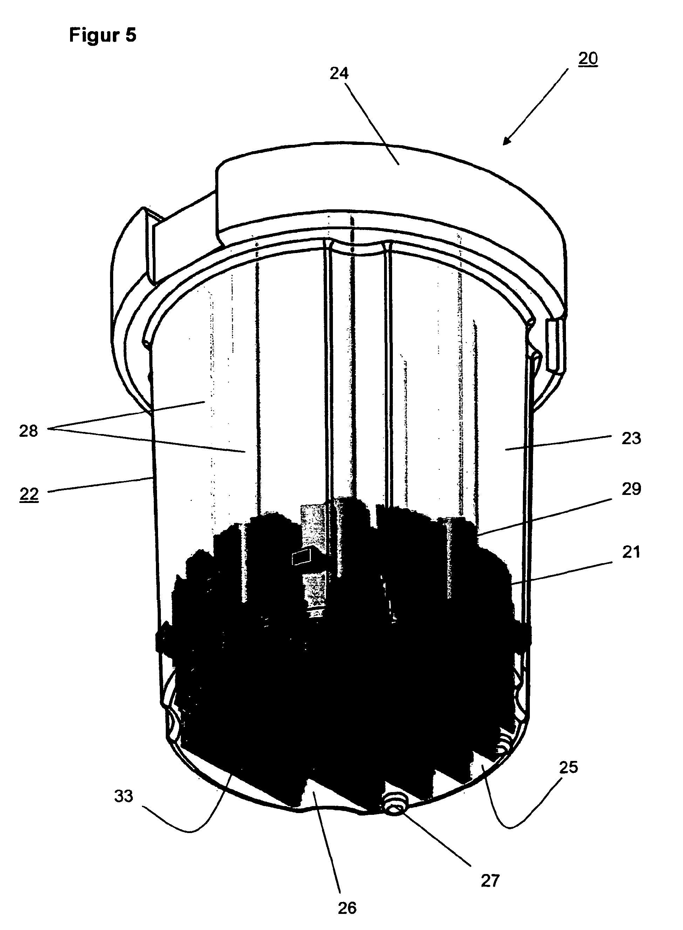

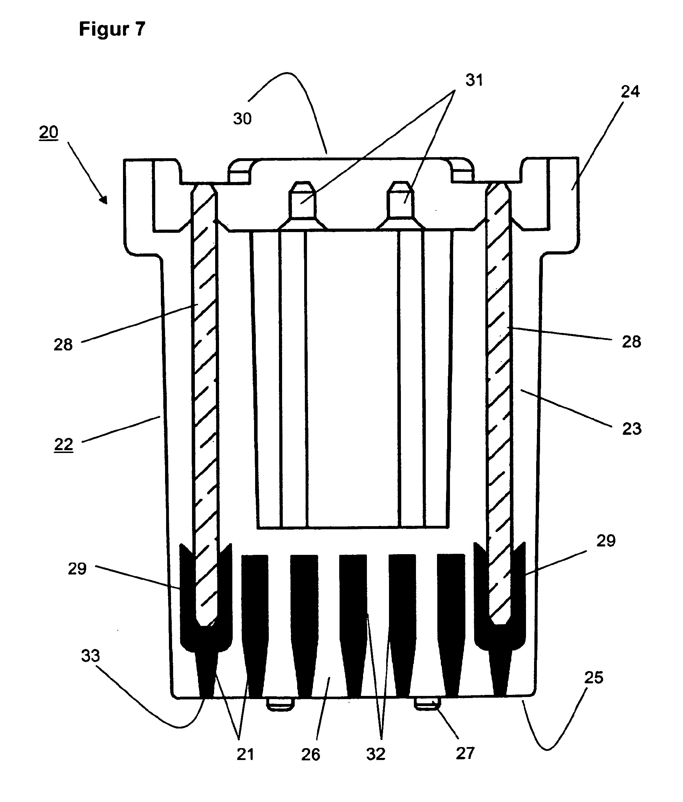

Figur 4 zeigt eine perspektivische Ansicht der Unterseite einer beispielhaften Ausführungsform einer erfindungsgemäßen Elektrodenanordnung 20. Die Elektrodenanordnung 20 umfasst sieben Elektroden 21, welche im Folgenden in Bezug auf die Figuren 5 und 7 noch näher beschrieben sind. Die Elektroden 21 sind in einer Halterung 22 angeordnet, die im Wesentlichen zylinderförmig ausgebildet ist. Die Halterung 22 umfasst einen Grundkörper 23 und einen am oberen Ende des Grundkörpers 23 angeordneten Randbereich 24, wobei der Außendurchmesser des Randbereichs 24 größer als der Außendurchmesser des Grundkörpers 23 ist, so dass der Randbereich 24 nach außen über den Grundkörper 23 hinausragt. Die Elektroden 21 sind zum größten Teil innerhalb des Grundkörpers 23 angeordnet und liegen mit ihrer unteren Stirnfläche 33 an der Unterseite 25 der Halterung 22 frei, so dass sie mit der Umgebung in Kontakt stehen. Die einzelnen Elektroden 21 sind jeweils durch ein isolierendes Material 26 elektrisch voneinander getrennt, wobei das isolierende Material 26 in diesem Ausführungsbeispiel den Raum zwischen den einzelnen Elektroden 21 vollständig ausfüllt. Das isolierende Material 26 zwischen den sich jeweils gegenüberliegenden Flächen der Elektroden 21 gewährleistet, dass beim Anlegen einer Spannung an die Elektroden kein Strom über den Raum zwischen den Elektroden 21 abfließen kann, wenn die Elektroden 21 in eine elektrisch leitende Flüssigkeit eingetaucht sind. Das isolierende Material 26 bewirkt vielmehr, dass beim Anlegen einer Spannung an die Elektroden 21 Strom über die Stirnflächen 33 der Elektroden 21 fließt und sich ein elektrisches Feld unterhalb der Unterseite 25 der Halterung 22 bildet. Da keine nennenswerten Ströme über den Raum zwischen den Elektroden 21 abfließen können, fällt die Spannung bei der Entladung eines Kondensators oder einer anderen nicht-permanenten Spannungsquelle nur langsam ab, so dass über die Zeit konstante und stabile Ströme fließen, die ein für die meisten biologischen Verfahren, beispielsweise die Elektrotransfektion, ausreichend starkes elektrisches Feld über die Zeit der Entladung erzeugen. Die Elektrodenanordnung 20 ist insbesondere dafür vorgesehen, in ein zumindest teilweise mit Flüssigkeit gefülltes Gefäß, beispielsweise ein Reaktionsbehältnis, eine Zellkulturschale oder ein "Well" einer Multiwell-Platte, eingesetzt zu werden, wobei dieses Gefäß eine Bodenfläche aufweist, an der lebende Zellen anhaften können. Die adhärenten Zellen auf der Bodenfläche des Gefäßes sind üblicherweise mit einer geeigneten Flüssigkeit, beispielsweise einem Zellkulturmedium oder einer an die gewünschte elektrische Behandlung adaptierten Lösung, bedeckt, wobei die Elektrodenanordnung 20 beim Einsetzen in das Gefäß zumindest einen Teil dieser Flüssigkeit verdrängt. Damit die Elektroden 21 mit ihren Stirnflächen 33 nicht direkt auf der Bodenfläche des Gefäßes und damit auf den Zellen aufliegen, weist die Unterseite 25 der Halterung 22 vier Abstandhalter 27 auf, die einen ausreichenden Abstand der Elektroden 21 zur Bodenfläche des Gefäßes gewährleisten.

Figur 5 zeigt eine perspektivische Ansicht der Elektrodenanordnung 20 gemäß

Figur 4, wobei in dieser Darstellung die innen liegenden Teile der Elektroden 21 sichtbar dargestellt sind. Es wird in dieser Darstellung deutlich, dass die Elektroden 21 im Wesentlichen plattenförmig ausgebildet sind, wobei die Dicke der Elektrodenplatten sich in Richtung der Unterseite 25 der Halterung 22 verringert. Die freiliegenden Stirnflächen 33 der Elektroden 21, die mit der Flüssigkeit in dem Gefäß in Kontakt stehen, sind also wesentlich schmaler als die innerhalb des Grundkörpers 23 angeordneten Teile der Elektroden 21. Dies hat den Vorteil, dass der Bereich unterhalb der jeweiligen Elektrode 21, innerhalb dessen eine effektive elektrische Behandlung der Zellen aufgrund des zu schwachen elektrischen Feldes nicht möglich ist, minimiert wird. Am gegenüberliegenden

Ende müssen die Elektroden 21 dagegen eine größere Dicke aufweisen, da sie hier zur Herstellung eines ausreichenden elektrischen Kontakts effektiv kontaktiert werden müssen. Der elektrische Kontakt zu der jeweils verwendeten Spannungsquelle wird hierbei im vorliegenden Ausführungsbeispiel über stiftförmige Kontaktelemente 28 hergestellt, die in verdickte Bereiche 29 der Elektroden 21 eingesetzt sind. Die Kontaktelemente 28 werden jeweils an ihrem dem Bereich 29 gegenüberliegenden Ende mittels einer geeigneten Kontaktvorrichtung elektrisch mit einer Spannungsquelle verbunden. Bei der Spannungsquelle kann es sich beispielsweise um einen oder mehrere Kondensatoren handeln, der bzw. die die kontrollierte Abgabe von Spannungsimpulsen ermöglicht bzw. ermöglichen. Die erzeugten Spannungsimpulse werden über die Kontaktelemente 28 an die Elektroden 21 weitergeleitet, so dass an der Unterseite der Elektroden 21, d. h. unterhalb der Unterseite 25 der Halterung 22, ein elektrisches Feld entsteht, welches aufgrund des isolierenden Materials 26 zwischen den Elektroden 21 auf den Raum zwischen den Zellen und der den Zellen zugewandten Seite der Elektroden 21 begrenzt bzw. fokussiert ist.

Die erfindungsgemäße Elektrodenanordnung 20 wird vorzugsweise im Spritzgussverfahren hergestellt. Dabei werden zunächst die Kontaktelemente 28 in ein geeignetes Spritzgusswerkzeug eingelegt und dann mit einem elektrisch isolierenden Polymer umspritzt. In einem zweiten Schritt wird dann ein elektrisch leitendes Polymer eingespritzt, das die Elektroden 21 bildet. Alternativ können die Elektroden auch aus einem Metall, vorzugsweise Aluminium, bestehen. Bei dieser Ausführungsform werden zunächst die Metallelektroden in das Spritzgusswerkzeug eingelegt und dann von einem elektrisch isolierenden Polymer umspritzt. Bei dieser Ausführungsform weisen die Metallelektroden vorzugsweise nach oben hinausragende Fortsätze auf, über welche die Elektroden elektrisch kontaktiert werden können.

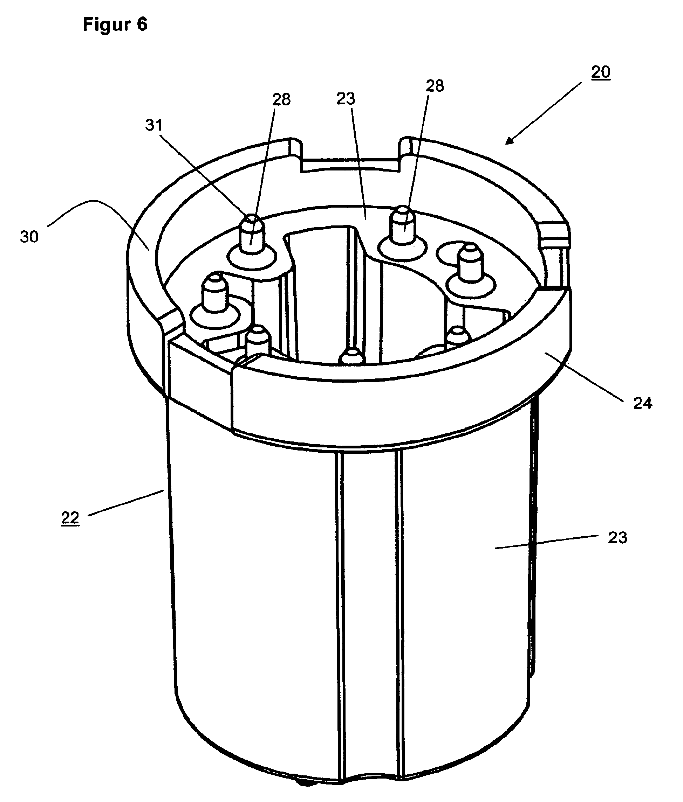

Figur 6 zeigt eine perspektivische Ansicht der Oberseite 30 der erfindungsgemäßen Elektrodenanordnung 20 gemäß Figur 4. Es wird hier deutlich, dass die Kontaktelemente 28 nach oben aus dem Grundkörper 23 herausragen. Die Kontaktelemente 28 sind also bis auf die freiliegenden Enden 31 vollständig von dem elektrisch isolierenden Material des Grundkörpers 23 umgeben. Über die freiliegenden Enden 31 können die Kontaktelemente 28 mittels einer geeigneten Vorrichtung elektrisch mit einer Spannungsquelle verbunden werden.

Figur 7 zeigt einen Längsschnitt durch die Elektrodenanordnung 20 gemäß den Figuren 4 bis 6. In dieser Darstellung wird deutlich, dass sich der Durchmesser der Elektroden 21 in Richtung der Unterseite 25 des Grundkörpers 23 verjüngt, so dass die Fläche unterhalb der Elektroden 21, innerhalb der sich nur ein unzureichendes elektrisches Feld ausbildet, minimiert wird. Am entgegengesetzten Ende der Elektroden 21 befindet sich der Bereich 29 mit vergrößerter Dicke, in den jeweils die Kontaktelemente 28 eingesetzt bzw. eingespritzt sind. Diese besonders vorteilhafte Ausgestaltung gewährleistet einen ausreichenden elektrischen Kontakt zwischen den Kontaktelementen 28 und den Elektroden 21, so dass eine effektive Weiterleitung der Spannungsimpulse von der Spannungsquelle bis zu den Elektroden 21 sichergestellt ist. Wenn die Elektrodenanordnung 20 in ein mit Flüssigkeit gefülltes Gefäß, an dessen Bodenfläche lebende Zellen anhaften, eingesetzt wird, sorgen die Abstandhalter 27 dafür, dass ein optimaler Abstand zwischen der Unterseite der Elektroden 21 und den zu behandelnden Zellen eingestellt wird. Da der Raum zwischen den einander jeweils gegenüber liegenden Flächen 32 der Elektroden 21 mit dem isolierenden Material 26 vollständig ausgefüllt ist, gelangt keine Flüssigkeit zwischen die Flächen 32 der Elektroden 21, so dass kein Strom über den Bereich zwischen den Flächen 32 der Elektroden 21 abfließen kann. Auf diese Weise wird beim Anlegen einer Spannung an die Elektroden 21 das elektrische Feld an der den Zellen zugewandten Seite der Elektroden 21 konzentriert und auf den Raum zwischen den Zellen und den Elektroden 21 begrenzt bzw. fokussiert. Auf diese Weise können die Zellen sehr effektiv und unter relativ geringem Energieaufwand behandelt werden. Ein weiterer Vorteil der Erfindung liegt darin, dass die Elektrodenanordnung 20 beim Einsetzen in das Gefäß einen Teil der Flüssigkeit verdrängt, da keine Zwischenräume zwischen den Elektroden 21 vorhanden sind. Aus diesem Grund muss das Gefäß nur mit einer geringen Menge Flüssigkeit gefüllt sein, wodurch für die Behandlung benötigte Lösungen und Substanzen eingespart und somit Kosten reduziert werden können.

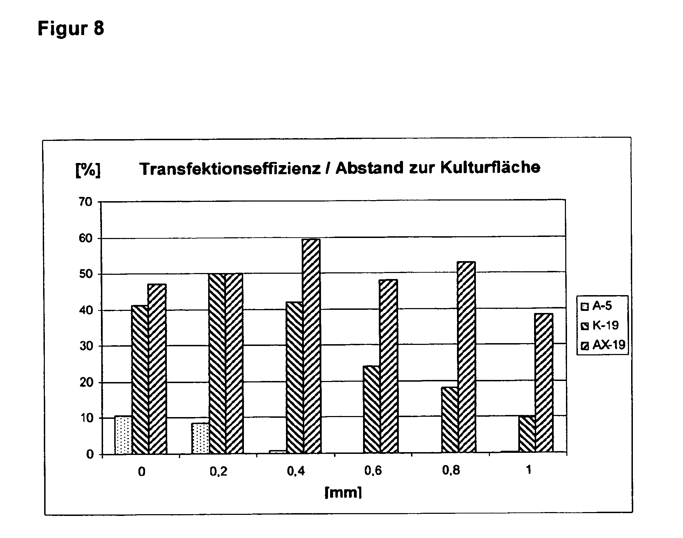

Figur 8 zeigt die Abhängigkeit der Transfektionseffizienz vom Abstand der Elektroden zu den zu behandelnden Zellen jeweils mit unterschiedlich starken Spannungsimpulsen. Transfektion bedeutet in diesem Zusammenhang das Einbringen von Nukleinsäuremolekülen (hier DNA) in lebende Zellen mittels elektrischer Spannungsimpulse. Während bei relativ hohen Spannungen (AX-19) nur eine geringe Abhängigkeit der Transfektionseffizienz vom Abstand zwischen den Elektroden und den Zellen vorliegt, zeigt sich bei schwachen Spannungsimpulsen (A-5), dass die Transfektionseffizienz um so größer wird, je geringer der Abstand zwischen den Elektroden und den Zellen ist. Mittelstarke Spannungsimpulse (K-19) zeigen dagegen ein Optimum bei mittelgroßen Abständen. Es wird also deutlich, dass der Abstand zwischen den Elektroden und den Zellen einen in Abhängigkeit von der Stärke der Spannungsimpulse mehr oder weniger großen Einfluss auf die Transfektionseffizienz hat.

### Bezugszeichenliste:

- 1: Elektrodenanordnung
- 2: Elektroden
- 3: Innenraum
- 4: Gefäß
- 5: Bodenfläche
- 6: Raum

- 10: Elektrodenanordnung
- 11: isolierendes Material
- 12: Elektroden
- 13: Innenraum
- 14: Gefäß
- 15: Bodenfläche
- 16: Fläche
- 17: Elektrodenanordnung
- 18: Elektroden
- 19: Isolierendes Material
- 20: Elektrodenanordnung
- 21: Elektroden
- 22: Halterung
- 23: Grundkörper
- 24: Randbereich
- 25: Unterseite
- 26: isolierendes Material
- 27: Abstandhalter
- 28: Kontaktelemente
- 29: Bereich
- 30: Oberseite
- 31: Ende
- 32: Fläche
- 33: Stirnfläche

## Patentansprüche

1. Elektrodenanordnung (10, 17, 20), insbesondere zur Beaufschlagung von adhärenten Zellen mit mindestens einem elektrischen Feld, die mindestens zwei Elektroden (12, 18, 21) umfasst, welche jeweils mindestens eine Fläche (16, 32) aufweisen, die der entsprechenden Fläche (16, 32) der jeweils anderen Elektrode (12, 18, 21) gegenüberliegend angeordnet ist, wobei zwischen den Flächen (16, 32) der Elektroden (12, 18, 21) zumindest teilweise ein elektrisch isolierendes Material (11, 19, 26) angeordnet ist, **dadurch gekennzeichnet, dass** die Flächen (16, 32) durch das isolierende Material (11, 19, 26) vollständig voneinander getrennt sind.

2. Elektrodenanordnung nach Anspruch 1, **dadurch gekennzeichnet, dass** mindestens drei, vorzugsweise mindestens 4 oder 5, insbesondere 6-12, Elektroden (12, 18, 21) vorgesehen sind.

3. Elektrodenanordnung nach Anspruch 1 oder 2, **dadurch gekennzeichnet, dass** die Elektroden (12, 18, 21) platten- oderstiftförmig ausgebildet sind.

4. Elektrodenanordnung nach einem der Ansprüche 1 bis 3, **dadurch gekennzeichnet, dass** die Flächen (16, 32) Seitenflächen planparallel angeordneter Elektrodenplatten sind.

5. Elektrodenanordnung nach einem der Ansprüche 1 bis 4, **dadurch gekennzeichnet, dass** der von den Flächen (16, 32) der Elektroden (12, 18, 21) begrenzte Raum zwischen den Elektroden (12, 18, 21) vollständig von dem isolierenden Material (11, 19, 26) ausgefüllt ist.

6. Elektrodenanordnung nach einem der Ansprüche 1 bis 5, **dadurch gekennzeichnet, dass** das isolierenden Material (11, 19, 26) ein thermoplastisches Polymer ist, vorzugsweise Polyvinylchlorid, Polystyrol, Polypropylen, Polyethylen und/oder Polykarbonat.

7. Elektrodenanordnung nach einem der Ansprüche 1 bis 6, **dadurch gekennzeichnet, dass** die Elektroden (12, 18, 21) aus Metall und/oder einem elektrisch leitenden Kunststoff bestehen.

8. Elektrodenanordnung nach einem der Ansprüche 1 bis 7, **dadurch gekennzeichnet, dass** die Elektrodenanordnung (10, 17, 20) an mindestens einer den Zellen zugewandten Seite mindestens einen Abstandhalter (27) aufweist.

9. Elektrodenanordnung nach einem der Ansprüche 1 bis 8, **dadurch gekennzeichnet, dass** die Elektrodenanordnung (10, 17, 20) zum Einsetzen in mindestens ein zumindest teilweise mit Flüssigkeit gefülltes Gefäß (14) vorgesehen ist, vorzugsweise ein Gefäß (14), an dessen Bodenfläche (15) lebende Zellen anhaften, und dass das isolierende Material (11, 19, 26) beim Einsetzen in das Gefäß (14) zumindest einen Teil der Flüssigkeit verdrängt.

10. Elektrodenanordnung nach einem der Ansprüche 1 bis 9, **dadurch gekennzeichnet, dass** die Elektroden (12, 18, 21) zumindest teilweise an der Unterseite (25) einer Halterung (22) angeordnet sind.

11. Elektrodenanordnung nach Anspruch 10, **dadurch gekennzeichnet, dass** die Halterung (22) derart ausgebildet ist, dass sie in ein Reaktionsgefäß eingesetzt oder auf dieses aufgesetzt werden kann, so dass die Elektroden (12, 18, 21) mit dem Innenraum des Reaktionsgefäßes in Kontakt stehen.

12. Elektrodenanordnung nach Anspruch 11, **dadurch gekennzeichnet, dass** das Reaktionsgefäß Teil einer Multiwell-Platte ist.

13. Verwendung der Elektrodenanordnung nach einem der Ansprüche 1 bis 12 zum Beaufschlagen von adhärenten Zellen mit mindestens einem elektrischen Feld, insbesondere zur Elektroporation von adhärenten Zellen, vorzugsweise in Form mindestens einer Eintauchelektrodenvorrichtung.

14. Verfahren zur Beaufschlagung von adhärenten Zellen mit mindestens einem elektrischen Feld, bei dem das elektrische Feld durch das Anlegen einer Spannung an mindestens zwei Elektroden (12, 18, 21) erzeugt wird, **dadurch gekennzeichnet, dass** das elektrische Feld an der den Zellen zugewandten Seite der Elektroden (12, 18, 21) dadurch konzentriert und/oder auf den Raum zwischen den Zellen und der den Zellen zugewandten Seite der Elektroden (12,18, 21) begrenzt wird, dass elektrisch isolierendes Material (11, 19, 26) zwischen den Elektroden (12, 18, 21) platziert wird, welches die einander zugewandten Flächen (16, 32) der Elektroden (12, 18, 21) vollständig voneinander trennt.

15. Verfahren nach Anspruch 14, **dadurch gekennzeichnet, dass** das elektrische Feld auf den Raum zwischen den Zellen und einer freiliegenden Stirnseite der Elektroden (12, 18, 21) begrenzt wird.

16. Verfahren nach Anspruch 14 oder 15, **dadurch gekennzeichnet, dass** die Elektroden (12, 18, 21) mit einer freiliegenden Stirnseite in mindestens ein Gefäß (14) eingebracht werden, an dessen Bodenfläche (15) die Zellen anhaften.

17. Verfahren nach einem der Ansprüche 14 bis 16, **dadurch gekennzeichnet, dass** die Wirkung des elektrischen Feldes auf die Zellen durch Einstellen des Abstands zwischen den Zellen und den Elektroden (12, 18, 21) optimiert wird.

## Claims

1. Electrode arrangement (10, 17, 20), in particular for applying at least one electric field to adherent cells, comprising at least two electrodes (12, 18, 21) which each include at least one area (16, 32) being disposed face to face with the corresponding area (16, 32) of the respective other electrode (12, 18, 21), wherein an electrically isolating material (11, 19, 26) is at least partially disposed between the areas (16, 32) of the electrodes (12, 18, 21), **characterized in that** the areas (16, 32) are completely separated from each other by the isolating material (11, 19, 26).

2. Electrode arrangement according to claim 1, **characterized in that** at least three, preferably at least 4 or 5, in particular 6-12, electrodes (12, 18, 21) are provided.

3. Electrode arrangement according to claim 1 or 2, **characterized in that** the electrodes (12, 18, 21) are formed like a plate or pin.

4. Electrode arrangement according to any one of claims 1 to 3, **characterized in that** the areas (16, 32) are lateral surfaces of electrode plates that are disposed coplanar.

5. Electrode arrangement according to any one of claims 1 to 4 **characterized in that** the space between the electrodes (12, 18, 21) defined by the areas (16, 32) of the electrodes (12, 18, 21) is completely filled by the isolating material (11, 19, 26).

6. Electrode arrangement according to any one of claims 1 to 5, **characterized in that** the isolating material (11, 19, 26) is a thermoplastic polymer, preferably polyvinylchloride, polystyrene, polypropylene, polyethylene and/or polycarbonate.

7. Electrode arrangement according to any one of claims 1 to 6, **characterized in that** the electrodes (12, 18, 21) are made of metal and/or an electrically conducting synthetic material.

8. Electrode arrangement according to any one of claims 1 to 7, **characterized in that** the electrode arrangement (10, 17, 20) includes at least one spacer (27) at at least one side facing the cells.

9. Electrode arrangement according to any one of claims 1 to 8, **characterized in that** the electrode arrangement (10, 17, 20) is provided for insertion into at least one container (14) being at least partially filled with a liquid, preferably a container (14) having a bottom area (15) to which living cells adhere, and that the isolating material (11, 19, 26) displaces at least a part of the liquid upon insertion into the container (14).

10. Electrode arrangement according to any one of claims 1 to 9, **characterized in that** the electrodes (12, 18, 21) are at least partially disposed at the underside (25) of a carrier (22).

11. Electrode arrangement according to claim 10, **characterized in that** the carrier (22) is designed such that it can be inserted into or placed onto a reaction vessel so that the electrodes (12, 18, 21) are exposed to the inner space of the reaction vessel.

12. Electrode arrangement according to claim 11, **characterized in that** the reaction vessel is part of a multiwall plate.

13. Use of the electrode arrangement according to any one of claims 1 to 12 for applying at least one electric field to adherent cells, in particular for electroporation of adherent cells, preferably in the form of at least one dipping electrode device.

14. Method for applying at least one electric field to adherent cells, in which the electric field is generated by applying a voltage to at least two electrodes (12, 18, 21), **characterized in that** the electric field is focused at the side of the electrodes (12, 18, 21) facing the cells and/or restricted to the space between the cells and the side of the electrodes (12, 18, 21) facing the cells by placing electrically isolating material (11, 18, 26) between the electrodes (12, 18, 21 which completely separates from each other the areas (16, 32) of the electrodes (12, 18, 21) being disposed face to face.

15. Method according to claim 14, **characterized in that** the electric field is restricted to the space between the cells and an exposed front side of the electrodes (12, 18, 21).

16. Method according to any one of claims 14 or 15, **characterized in that** an exposed front side of the electrodes (12, 18, 21) is inserted into at least one container (14) having a bottom area (15) to which the cells adhere.

17. Method according to any one of claims 14 to 16, **characterized in that** the effect of the electric field on the cells is optimized by adjusting the distance between the cells and the electrodes (12, 18, 21).

## Revendications

1. Agencement d'électrodes (10, 17, 20), en particulier pour appliquer des cellules adhérentes avec au moins un champ électrique qui comprend au moins deux électrodes (12, 18, 21) qui présentent respectivement au moins une surface (16, 32) qui est disposée opposée à la surface correspondante (16, 32) de l'autre électrode (12, 18, 21) possible, un matériau électro-isolant (11, 19, 26) étant au moins partiellement disposé entre les surfaces (16, 32) des électrodes (12, 18, 21) **caractérisé en ce que** les surfaces (16, 32) sont complètement séparées l'une de l'autre par le métériau isolant (11, 19, 26).

2. Agencement d'électrodes selon la revendication 1 **caractérisé en ce qu'**au moins trois, de préférence au moins 4 ou 5, en particulier 6-12 électrodes (12, 18, 21) sont prévues.

3. Agencement d'électrodes selon la revendication 1 ou 2 **caractérisé en ce que** les électrodes (12, 18, 21) sont constituées en forme de plaques ou de tiges.

4. Agencement d'électrodes selon une quelconque des revendications 1 à 3 **caractérisé en ce que** les surfaces (16, 32) sont des surfaces latérales de plaques d'électrodes montées parallèlement planes.

5. Agencement d'électrodes selon une quelconque des revendications 1 à 4 **caractérisé en ce que** l'espace limité par les surfaces (16, 32) des électrodes (12, 18, 21) entre les électrodes (12, 18, 21) est complètement rempli du matériau isolant (11, 19, 26).

6. Agencement d'électrodes selon une quelconque des revendications 1 à 5 **caractérisé en ce que** le matériau isolant (11, 19, 26) est un polymère thermoplastique, de préférence du chlorure de polyvinyle, du polystyrène, du polypropylène, du polyéthylène et/ou du polycarbonate.

7. Agencement d'électrodes selon une quelconque des revendications 1 à 6 **caractérisé en ce que** les électrodes (12, 18, 21) sont composées de métal et/ou d'un plastique électroconducteur.

8. Agencement d'électrodes selon une quelconque des revendications 1 à 7 **caractérisé en ce que** l'agencement d'électrodes (10, 17, 20) présente au moins une entretoise (27) sur un côté tourné au moins vers une cellule.

9. Agencement d'électrodes selon une quelconque des revendications 1 à 8 **caractérisé en ce que** l'agencement d'électrodes (10, 17, 20) est prévu pour introduction au moins dans une cuve (14) rempli au moins partiellement de liquide, de préférence une cuve (14) à la surface de fond (15) de laquelle adhèrent des cellules vivantes et **en ce que** le matériau isolant (11, 19, 26) déplace au moins une partie du liquide lors de l'introduction dans la cuve (14).

10. Agencement d'électrodes selon une quelconque des revendications 1 à 9 **caractérisé en ce que** les électrodes (12, 18, 21) sont disposées au moins en partie sur la face inférieure (25) d'un support (22).

11. Agencement d'électrodes selon la revendication 10 **caractérisé en ce que** le support (22) est constitué de telle sorte qu'il peut être introduit dans une cuve de réaction ou posé sur celle-ci de sorte que les électrodes (12, 18, 21) se trouvent en contact avec le compartiment intérieur de la cuve de réaction.

12. Agencement d'électrodes selon la revendication 11 **caractérisé en ce que** la cuve de réaction est une partie d'une plaque Multiwell.

13. Utilisation de l'agencement d'électrodes selon une quelconque des revendications 1 à 12 pour appliquer des cellules adhérentes avec au moins un champ électrique, en particulier pour l'électroporation de cellules adhérentes, de préférence sous la forme d'au moins un dispositif d'électrodes immergées.

14. Procédé pour appliquer des cellules adhérentes avec au moins un champ électrique, pour lequel le champ électrique est produit par l'application d'une tension sur au moins deux électrodes (12, 18, 21) **caractérisé en ce que** le champ électrique est de ce fait concentré sur le côté des électrodes (12, 18, 21) tourné vers les cellules et/ou est limité au côté des électrodes (12, 18, 21) tourné vers les cellules, **en ce que** le matériau électro-isolant (11, 19, 26) est placé entre les électrodes (12, 18, 21) lequel sépare complètement l'une de l'autre les surfaces (16, 32) des électrodes (12, 18, 21) tournées l'une vers l'autre.

15. Procédé selon la revendication 14 **caractérisé en ce que** le champ électrique est limité à l'espace situé entre les cellules et un côté avant libre des électrodes (12, 18, 21).

16. Procédé selon la revendication 14 ou 15 **caractérisé en ce que** les électrodes (12, 18, 21) sont introduites avec une face avant libre dans au moins une cuve (14) sur la surface de fond (15) de laquelle adhérent les cellules.

17. Procédé selon une quelconque des revendications 14 à 16 **caractérisé en ce que** l'effet du champ électrique est optimisé sur les cellules en ajustant l'intervalle entre les cellules et les électrodes (12, 18, 21).
